# NEUE EUROPÄISCHE PATENTSCHRIFT

(11) **EP 1 897 579 B2**
(45) Veröffentlichungstag und Bekanntmachung des Hinweises auf die Entscheidung über den Einspruch: **16.10.2013**
(45) Hinweis auf die Patenterteilung: 25.11.2009
(21) Anmeldenummer: 07014618.8
(22) Anmeldetag: 25.07.2007
(51) Int. Cl.: A61M 25/00

(54) **Katheter-Set**
Catheter set
Ensemble de cathéter

(30) Priorität: 18.04.2007 DE 102007018276; 06.09.2006 DE 202006013663 U
(43) Veröffentlichungstag der Anmeldung: 12.03.2008
(73) Patentinhaber: Medical Service GmbH, 75378 Bad Liebenzell (DE)
(72) Erfinder: Burkert, Ralf, 75378 Bad Liebenzell (DE); Bobzien, Sonja, 71263 Weil der Stadt (DE)
(74) Vertreter: Grünecker, Kinkeldey, Stockmair & Schwanhäusser

(56) Entgegenhaltungen:
- EP-A- 0 913 164
- DE-A1- 2 317 839
- DE-U1- 20 021 431
- DE-U1-202005 009 946
- GB-A- 2 319 507
- US-A- 5 593 394
- US-A1- 2002 156 440
- US-A1- 2003 060 807
- US-A1- 2004 074 794
- US-A1- 2005 043 715
- US-A1- 2005 070 882
- US-A1- 2005 109 648

## Beschreibung

Die Erfindung betrifft ein Katheter-Set, umfassend eine aus einem folienartigen Material hergestellte Verpackung, in der ein Reservoir mit einem Aktivierungsmittel und/oder einem Gleitmittel vorgesehen ist, und einen Blasenkatheter mit einem flexiblen Katheterschaft und einer Katheterspitze, wobei die Verpackung einen im Wesentlichen röhrenförmigen Katheteraufnahmeraum aufweist, in den der Blasenkatheter eingelegt ist. An dem der Katheterspitze zugeordneten Ende des Katheteraufnahmeraums ist eine Austrittsstelle für den Blasenkatheter vorgesehen und das Reservoir ist an einer Längsseite des Katheteraufnahmeraums am Katheteraufnahmeraum angeordnet.

Ein derartiges Katheter-Set, wie es beispielsweise aus der DE 20 2005 009 946 U1 bekannt geworden ist, erlaubt eine sterile Verpackung eines Blasenkatheters, der zum Gebrauch durch die Austrittsstelle aus der Verpackung geführt wird. Der Blasenkatheter tritt mit der Katheterspitze voran aus der Verpackung und wird direkt in die Harnröhre zur Harnblase eines Patienten geführt. Durch die gebrauchsfertige Lage des Blasenkatheters innerhalb des Katheteraufnahmeraums der Primärverpackung und durch die Flexibilität des Verpackungsmaterials wird ein unmittelbarer Kontakt zwischen dem Blasenkatheter und den ihn haltenden bzw. führenden Händen vermieden. Um mittels des Katheters abgeführten Urin aufzufangen, weist das Katheter-Set einen an den Katheteraufnahmeraum anschließenden Urinaufnahmeraum auf. Der Urin wird durch den Katheteraufnahmeraum zum Urinaufnahmeraum geleitet.
In der Verpackung ist ein von der Verpackung ausgebildeter Reservoiraufnahmeraum seitlich neben dem Katheteraufnahmeraum mittig angeordnet, in dem ein Reservoir mit z.B. Oberflächenaktivierungsmittel vorgehalten ist. Dieses Reservoir wird vor dem Einführen des Katheters in die Harnröhre zur Harnblase eines Patienten aufgedrückt oder geöffnet, so dass das Oberflächenaktivierungsmittel auf der Oberfläche des Blasenkatheters zu dessen Benetzung verteilt werden kann um dessen Gleiteigenschaften zu verbessern.

Ein derartiges Katheter-Set, wie es in dem Oberbegriff nach Anspruch 1 beschrieben ist, ist auch aus dem Dokument US 2003/060807 bekannt.

Bei bekannten Katheter-Sets läuft das Oberflächenaktivierungsmittel nach dem Öffnen des Reservoirs ungerichtet in den Katheteraufnahmeraum. Handelt es sich bei dem Oberflächenaktivierungsmittel um eine Flüssigkeit, so können Teile davon relativ leicht in den Urinaufnahmeraum abfließen und gehen deshalb für die Oberflächenaktivierung verloren. Handelt es sich bei dem Oberflächenaktivierungsmittel um ein Gel, so ist eine gleichmäßige Oberflächenbenetzung mit dem Gel nur durch sorgfältiges Verteilen des Gels um den Katheter herum, z.B. mittels eines schiebenden Drückens im gesamten Katheteraufnahmeraum, möglich. Da eine gleichmäßige und vollständige Oberflächenbenetzung mit Oberflächenaktivierungsmittel für eine gute Gleitfähigkeit beim Einführen des Katheters Voraussetzung ist, ist die Handhabug bei einer Katheterapplikation bei bekannten Katheter-Sets durch die genannten Probleme erschwert.

Der Erfindung liegt die Aufgabe zugrunde, ein Katheter-Set bereitzustellen, welches die Nachteile des Standes der Technik vermeidet, wobei insbesondere die Benetzung der Katheteroberfläche mit einem Oberflächenaktivierungsmittel vereinfacht werden soll.

Diese Aufgabe wird durch das Katheter-Set des unabhängigen Anspruchs gelöst. Die abhängigen Ansprüche stellen bevorzugte Ausführungsformen der Erfindung dar.

Erfindungsgemäß ist in der Verpackung eine Aktivierungs- und/oder Gleitmittelführung ausgebildet, die , nach einem Öffnen des Reservoirs, eine in Richtung der Katheterspitze gerichtete Benetzung der Oberfläche des Blasenkatheters mit Aktivierungsmittel und/oder Gleitmittel aus dem Reservoir unterstützt. Diese Aktivierungs- und/oder Gleitmittelführung ist in Richtung der Katheterspitze gerichtet und verbindet einen in der Verpackung ausgebildeten Reservoiraufnahmeraum, in dem das Reservoir angeordnet ist, mit dem Katheteraufnahmeraum.

Im Falle der Verwendung einer wasserhaltigen Flüssigkeit als Aktivierungsmittel weist der Blasenkatheter bevorzugt eine hydrophile Oberflächenbeschichtung auf, um eine Aktivierung der Beschichtung des Blasenkatheters zu ermöglichen. Im Falle der Verwendung z.B. eines Gleitmittels erfolgt vor Applikation des Blasenkatheters eine Gleitmittelbenetzung der Oberfläche des Blasenkatheters. Durch eine entsprechende Beschichtung werden die Gleiteigenschaften des Blasenkatheters verbessert, so dass dieser weitgehend schmerz- und irritationsfrei in den Körper des Patienten eingeführt werden kann. Mit Hilfe des Aktivierungsmittels, beispielsweise einer sterilen Kochsalzlösung, wird eine hydrophile Oberflächenbeschichtung des Blasenkatheters aktiviert, mit Hilfe eines Gleitmittels, wie einem Gel, wird der Blasenkatheter benetzt und/oder aktiviert, wodurch die gewünschte Gleitfähigkeit des Blasenkatheters erreicht wird. Dadurch, dass mittels der Aktivierungs- und/oder Gleitmittelführung die Benetzung der Oberfläche des Blasenkatheters in Richtung der Katheterspitze gerichtet vorgenommen wird, kann mit weniger Aktivierungs- und/oder Gleitmittel ausgekommen werden. Das Aktivierungs- und/oder Gleitmittel fließt aus dem Reservoir direkt Richtung Katheterspitze, bzw. kann im Falle der Verwendung eines Gleitmittels problemlos zur Katheterspitze geschoben werden. Die vollständige Benetzung des vorderen Katheterteils, d.h. des katheterspitzennahen Teils des Blasenkatheters, wird vereinfacht und unterstützt. Da üblicherweise der Blasenkatheter nicht vollständig in die Harnröhre eingeschoben wird, sondern hauptsächlich der genannte vordere Katheterteil, reicht die Benetzung des vorderen Teils bereits zur Applikation des Blasenkatheters aus.

Die Aktivierungs- und/oder Gleitmittelführung its als eine vom Reservoiraufnahmeraum schräg zum Katheteraufnahmeraum in Richtung der Katheterspitze geneigte, bevorzugt von einer Foliennaht ausgebildete, Flüssigkeitsführung ausgeführt. Derart lässt sich die Aktivierungs- und/oder Gleitmittelführung einfach durch Verbinden der die Verpackung ausbildenden Schichten des folienartigen Materials herstellen. Im Falle der Verwendung von Kunststofffolie zur Herstellung der Verpackung kann diese Foliennaht einfach als Schweißnaht ausgeführt sein.

Wenn das Reservoir in einem Bereich in der Mitte der Längsausdehnung des Katheteraufnahmeraums in der Verpackung angeordnet ist, kann das Aktivierungsmittel und/oder das Gleitmittel leichter im Bereich der Oberfläche des Blasenkatheters verteilt werden, der in die Harnröhre einzuführen ist, als bei einer Anordnung z.B. am katheterspitzenabgewandten Ende des Katheters. Eine derartige Anordnung des Reservoirs ist insbesondere dann vorteilhaft, wenn der Katheteraufnahmeraum in Längsrichtung der Verpackung verläuft und die Verpackung im Wesentlichen rechteckförmig ausgebildet ist. Insbesondere wenn ein Gel verwendet wird, kann das Gel dann aus dem Reservoir einfach in den katheterspitzennahen Bereich des Katheteraufnahmeraums gedrückt werden. Da der Katheter mit seiner gesamten in die Harnröhre einzuführenden Länge durch diesen Bereich durchgeschoben wird, wird die Oberfläche des Katheters dadurch gleichmäßig mit dem Gel überzogen. Der Bereich in der Mitte der Längsausdehnung des Katheteraufnahmeraums kann z.B. im mittleren Drittel der Gesamtlänge der Verpackung liegen. Wenn am Reservoir oder in der Verpackung ein in den Katheteraufnahmeraum führender Kanal oder eine derartige Öffnung für das Aktivierungsmittel und/oder das Gleitmittel vorgesehen ist, sollten diese in dem Bereich in der Mitte der Längsausdehnung des Katheteraufnahmeraums angeordnet sein.

Bei einem erfindungsgemäßen Katheter-Set können in zumindest einem Längenabschnitt des Katheteraufnahmeraums Führungselemente an der Außenoberfläche der Verpackung vorgesehen sein, wobei die Führungselemente in Längsrichtung des Katheteraufnahmeraums der Verpackung dem Verlauf des Katheteraufnahmeraums zumindest teilweise folgen.
Die Führungselemente sind entlang der länglichen Ausdehnung des Katheteraufnahmeraums, mit anderen Worten entlang der axialen Erstreckung des Katheterschafts, angeordnet. Zum Aus- bzw. Einführen des Blasenkatheters aus der Verpackung greift ein Benutzer mit beiden Händen außen an dem Katheteraufnahmeraum an, fixiert mit einer Hand die Verpackung am Katheterschaft und schiebt mit der anderen Hand den Blasenkatheter aus der Verpackung. Durch die Führungselemente wird die Griffigkeit der üblicherweise glatten Außenoberfläche der Verpackung erhöht und ein Ab- bzw. Verrutschen der den Blasenkatheter greifenden Finger vermieden. Auf diese Weise wird bei dem erfindungsgemäßen Katheter-Set bzw. Katheter-System ein vereinfachtes und erleichtertes Führen des flexiblen Katheterschafts sichergestellt. Durch die verbesserte Handhabbarkeit des Blasenkatheters ist die Hygiene beim Gebrauch des erfindungsgemäßen Katheter-Sets im Vergleich zu herkömmlichen Katheter-Sets erhöht, und das Ein- und/oder Ausführen eines Katheters aus der Verpackung wird erheblich erleichtert.
Bei einem sehr gleitfähigen Blasenkatheter wirken sich die Führungselemente besonders vorteilhaft aus, da durch die friktionsarme Oberfläche des Blasenkatheters die Reibung zwischen dem Blasenkatheter und dem ihn umgebenden Katheteraufnahmeraum verringert ist und somit ein indirektes Verschieben des Blasenkatheters in oder aus der Verpackung heraus mit den erfindungsgemäßen Führungselementen erheblich erleichtert wird.

Das Material der Verpackung ist in der Regel ein Kunststoff; es ist jedoch auch denkbar, ein Leichtmetall oder ein beschichtetes Gewebe oder Kombinationen davon einzusetzen. Durch Verkleben, Verschweißen und/oder Vernähen des folienartigen Materials erhält die Verpackung eine flächige Gestalt. Um eine sterile Lagerung bzw. eine aseptische Handhabung des Blasenkatheters sicherzustellen, ist der Katheteraufnahmeraum an der Austrittsstelle verschlossen. Vor, bzw. an der Austrittsstelle kann eine Sollbruchnaht vorgesehen sein, die vor oder beim Ausführen des Blasenkatheters durchbrochen wird. Ein vor dem Austritt des Blasenkatheters zu öffnendes Verschlusselement, z.B. eine Kappe oder dergleichen, ist dann vom Material der Verpackung, z.B. Kunststofffolie, mit umschlossen.

In einer bevorzugten Ausführungsform des erfindungsgemäßen Katheter-Sets sind die Führungselemente im Bereich der Austrittsstelle vorgesehen. Beim Aus- bzw. Einführen des Blasenkatheters greift ein Benutzer die Verpackung typischerweise nahe der Austrittsstelle an, um den Blasenkatheter in kontrollierter Weise in den Körper des Patienten einzuführen, so dass sich die Führungselemente in diesem Bereich als besonders vorteilhaft erweisen.

In einer weiteren bevorzugten Ausführungsform des erfindungsgemäßen Katheter-Sets erstrecken sich die Führungselemente über die gesamte Länge des Katheteraufnahmeraums. In dieser Ausführungsform sind die Führungselemente entlang des gesamten Katheteraufnahmeraums und folglich entlang des gesamten in der Verpackung aufgenommenen Blasenkatheters vorgesehen, wodurch die Handhabbarkeit des erfindungsgemäßen Katheter-Sets in jeder Aus- bzw. Einführstellung des Blasenkatheters verbessert und erleichtert ist.

Vorteilhafterweise sind die Führungselemente äquidistant, bevorzugt mit fingerbreitem Abstand, angeordnet. Das den Blasenkatheter umgebende Verpackungsmaterial wird durch Zurückschieben entlang der axialen Erstreckung des Katheterschafts zusammengedrückt bzw. zusammengerafft und ermöglicht so das Vorwärtsschieben des Blasenkatheters. Die in regelmäßigen Abständen angeordneten Führungselemente erleichtern das Verschieben der Verpackung entlang des Katheterschafts.

In einer bevorzugten Ausführungsform der Erfindung sind die Führungselemente an der Oberseite und der Unterseite der Verpackung vorgesehen. Durch die beidseitige Anordnung von Führungselementen werden alle den Katheterschaft umgreifenden Finger eines Benutzers gegen Verrutschen gesichert und somit das Ein- bzw. Ausführen des Blasenkatheters aus der Verpackung weiter erleichtert.

Die Führungselemente sind bevorzugterweise als Raffungen, Riffelungen, Noppen und/oder Antirutschelemente ausgebildet. Eine derartige Ausbildung der Führungselemente aus dem folienartigen Material der Verpackung vereinfacht die Herstellung des erfindungsgemäßen Katheter-Sets. Es ist jedoch auch denkbar, die Führungselemente nachträglich, beispielsweise durch Aufkleben oder Beflocken, an der Verpackung anzubringen.

Besonders bevorzugt ist bei einem erfindungsgemäßen Katheter der Katheteraufnahmeraum Teil eines Urinaufnahmeraums. Weiter ist an der Austrittsstelle ein wiederverschließbares Verschlusselement vorgesehen. D.h. die Austrittsstelle ist durch das Verschlusselement wiederverschließbar.
Bei einem derartigen Katheter-Set dient die Verpackung für den Blasenkatheter zugleich als Sammel- bzw. Aufnahmebeutel für den durch den Blasenkatheter abgeleiteten Urin. An dem der Katheterspitze gegenüberliegenden Ende des Blasenkatheters ist zweckmäßigerweise ein Blockierelement vorgesehen, welches ein vollständiges Herausziehen des Blasenkatheters aus der Verpackung verhindert und während des Katheterisierungsvorgangs den Katheteraufnahmeraum bzw. die Austrittsstelle nach außen abdichten kann. Dazu ist das Blockierelement in seiner äußeren Abmessung an die Abmessung der Austrittsstelle derart angepasst, dass diese mit dem Blockierelement als eine Art Stöpsel verschließbar ist, so dass während des Katheterisierungsvorgangs kein Urin ungewollt seitlich neben dem Katheter aus dem Katheteraufnahmeraum fließen kann.
Beim typischen Einsatz des erfindungsgemäßen Katheter-Sets wird der Blasenkatheter aus der Verpackung geschoben und in die Harnblase des Patienten eingeführt. Der Urin fließt während des Katheterisierungsvorgangs direkt in den Urinaufnahmeraum, mit anderen Worten in die Verpackung, und der Blasenkatheter wird anschließend aus dem Körperhohlorgan zurückgezogen und in die Verpackung geschoben. Abschließend wird die Austrittsstelle mittels des Verschlusselements wieder verschlossen. Durch das Verschlusselement kann die Verpackung nach Rückführung des Blasenkatheters wieder dicht verschlossen werden und die Verpackung samt des verwendeten Blasenkatheters und des in ihr gespeicherten Urins entsorgt werden. Da das Verschlusselement an der Austrittsstelle angeordnet ist, kann nach dem Katheterisierungsvorgang kein Urin aus dem Katheteraufnahmeraum austreten. Weiter kann das erfindungsgemäße Katheter-Set hinsichtlich seiner Gesamtabmessung relativ klein ausgeführt werden, da der Katheterraum gesamt oder teilweise zugleich als Teil des Urinaufnahmeraums genutzt wird.

Bevorzugt weist der Katheteraufnahmeraum eine an die Austrittsstelle angrenzende bzw. diese ausbildende Führungshülse auf. Beim Ausführen durchtritt der Blasenkatheter die Führungshülse und wird durch diese in seiner Austrittsrichtung stabilisiert. Die Führungshülse dient aufgrund dieser Richtungsstabilisierung als Einführhilfe beim Einführen des Blasenkatheters in ein Körperhohlorgan. Das Verschlusselement kann dabei als Verschlussklappe an der Führungshülse ausgebildet sein. Eine derartige Verschlussklappe lässt sich einfach durch Aufklappen öffnen und durch Zuklappen wieder verschließen. Das Verschlusselement kann dabei nicht verlorengehen, da die Klappe beweglich am Verschlusselement befestigt ist.

Dabei sind die Verschlussklappe und die Führungshülse sehr vorteilhaft einstückig aus Kunststoff hergestellt, wobei die Verschlussklappe und die Führungshülse über mindestens einen flexiblen Kunststoffstreifen miteinander verbunden sind. Diese Ausführungsform der Verschlussklappe mit der Führungshülse ist sehr einfach und kostengünstig herstellbar.

Vorteilhaft weist die Führungshülse an ihrer Innenoberfläche in Katheteraustrittsrichtung verlaufende Führungsrippen auf, so dass die in der Regel mit einem kleineren Durchmesser als der Innendurchmesser der Führungshülse ausgeführte Katheterspitze noch besser geführt wird. Insbesondere wenn eine Aktivierung der Katheteroberfläche mittels eines Gels als Gleitmittel vor dessen Applikation vorgesehen ist, wird durch die Führungsrippen eine gleichmäßige Verteilung des Gleitmittels auf der Katheteroberfläche gewährleistet. Insbesondere wird ein Abstreifen des Gleitmittels von Teilen der Katheteroberfläche durch die Führungshülse vermieden.

Zum besseren Abdichten beim Verschließen der Führungshülse mittels des Verschlusselements können die Führungshülse und/oder das Verschlusselement eine umlaufende Dichtlippe aufweisen.

In einer bevorzugten Ausführungsform des erfindungsgemäßen Katheter-Sets sind in der Verpackung eine oder mehrere Grifföffnungen ausgebildet. Durch die Grifföffnungen wird die Handhabung des Katheter-Sets weiter erleichtert, beispielsweise kann das Katheter-Set während des Katheterisierungsvorgangs befestigt, beispielsweise an einem Ständer oder Haken aufgehängt werden.

Wenn in einem an die Austrittsstelle angrenzenden Bereich des Katheteraufnahmeraums eine Katheteraufnahmeraumverbreiterung vorgesehen ist, eignet sich der Katheteraufnahmeraum besonders gut für die Aufnahme eines Tiemannkatheter als Katheter des Katheter-Sets.
Ein Tiemannkatheter besitzt eine gebogene konisch zulaufende Katheterspitze. Durch seine Form eignet er sich besonders zur Katheterisierung der männlichen Harnblase, da die Spitze den Katheter besser durch die Harnröhre führt.

Es ist darüber hinaus denkbar, in den Katheteraufnahmeraum einen Katheter zur Entleerung und/oder Spülung eines beliebigen Körperhohlorgans, insbesondere zum Ableiten einer beliebigen Körperflüssigkeit, einzulegen. Ebenfalls lassen sich die Führungselemente der Verpackung zum verbesserten Auspacken eines jeden Katheters nutzen und die erfindungsgemäße Ausbildung der Verpackung beschränkt sich nicht auf Blasenkatheter.

Weitere Vorteile und Merkmale der Erfindung ergeben sich aus der Beschreibung und den Figuren der Zeichnung. Ebenso können die vorstehend genannten und die noch weiter aufgeführten Merkmale erfindungsgemäß jeweils einzeln oder in beliebigen Kombinationen miteinander verwendet werden. Das erfindungsgemäße Katheter-Set ist in einem Ausführungsbeispiel in den Figuren gezeigt. Die in den Figuren gezeigten Merkmale sind rein schematisch und nicht maßstäblich zu verstehen. Es zeigt:
- Figur 1: eine Draufsicht eines erfindungsgemäßen Katheter-Sets;
- Figuren 2a und 2b: jeweils einen vergrößerten Ausschnitt eines erfindungsgemäßen Katheter-Sets; und
- Figuren 3a und 3b: das als Verschlussklappe ausgebildete und an der Führungshülse angeformte Verschlusselement des erfindungsgemäßen Katheter-Sets aus Figur 1; und
- Figur 4: eine weitere Ausführungsform des Katheter-Sets.

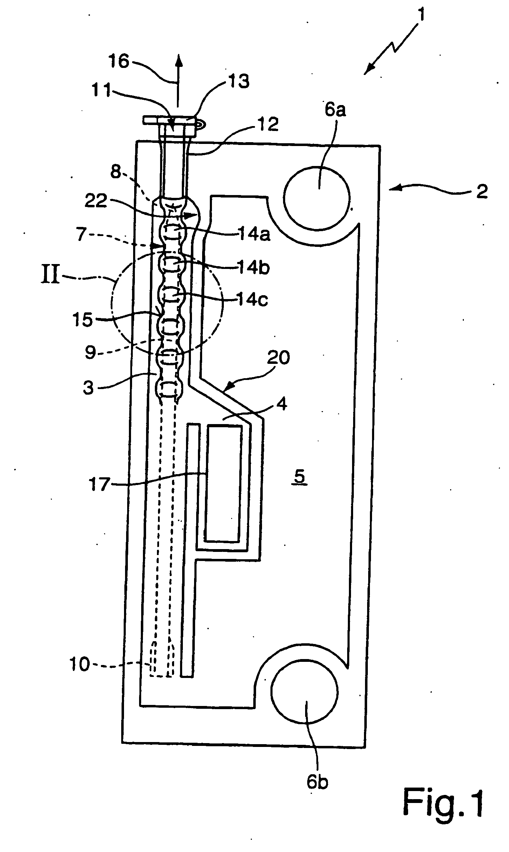

In Figur 1 ist in Draufsicht ein Katheter-Set 1 gezeigt, dessen Gestalt durch eine im Wesentlichen rechteckförmige Verpackung 2 bestimmt wird. Die Verpackung 2 besteht aus zwei miteinander verbundenen Lagen einer Kunststofffolie, in der ein länglicher, kanalartiger Katheteraufnahmeraum 3, ein Reservoiraufnahmeraum 4 und ein Urinaufnahmeraum 5 ausgebildet sind.

Der Katheteraufnahmeraum 3 verläuft in Längsrichtung der Verpackung 2. Der Reservoiraufnahmeraum 4 ist über den Katheteraufnahmeraum 3 mit dem Urinaufnahmeraum 5 verbunden und in einem Bereich in der Mitte der Längsausdehnung des Katheteraufnahmeraums 3, bzw. angrenzend an die Mitte des Katheteraufnahmeraums 3, angeordnet.
An beiden schmalseitigen Enden der Verpackung 2 sind als kreisförmige Löcher ausgebildete Grifföffnungen 6a und 6b vorgesehen. In den Katheteraufnahmeraum 3 ist ein Blasenkatheter 7 eingelegt. Der Blasenkatheter 7 besteht aus einer Katheterspitze 8, einem langgestreckten Katheterschaft 9 und einem gegenüber dem Katheterschaft 9 verbreiterten Blockierelement 10. Der Katheteraufnahmeraum 3 wird am Rand der Verpackung 2 durch eine Austrittsstelle 11 begrenzt, auf die die Katheterspitze 8 des eingelegten Katheters 7 zeigt. An die Austrittsstelle 11 grenzt eine Führungshülse 12 an, bzw. die Austrittsstelle 11 wird von der Führungshülse 12 ausgebildet. In die Führungshülse 12 mündet der kanalartige Katheteraufnahmeraum 3. An der Führungshülse 12 ist ein als Verschlussklappe ausgebildetes Verschlusselement 13 vorgesehen, durch welches das durch den Katheteraufnahmeraum 3, den Reservoiraufnahmeraum 4 und den Urinaufnahmeraum 5 definierte Aufnahmevolumen der Verpackung 2 verschlossen ist. Der Blasenkatheter 7 ist über die Verpackung 2 dauerhaft steril verpackt.
Der Reservoiraufnahmeraum 4 kann auch am unteren Ende des Katheteraufnahmeraums 3, d.h. im Bereich dessen austrittsstellenfernen Endes, angeordnet sein. Dann könnte der Katheteraufnahmeraum 3 über den Reservoiraufnahmeraum 4 mit dem Urinaufnahmeraum 5 verbunden sein.

Zum vereinfachten Ein- und Ausführen des Blasenkatheters 7 sind entlang der länglichen Erstreckung des Katheteraufnahmeraums 3 Führungselemente 14a, 14b, 14c, ... an einer Außenoberfläche 15 der Verpackung 2 vorgesehen.
Durch die reihenförmig angeordneten Führungselemente 14a, 14b, 14c, ... , die als noppenartige Raffungen an der Außenoberfläche 15 ausgebildet sind, wird das Herausführen bzw. Herauslösen des Blasenkatheters 7 aus der Verpackung 2 in Ausführrichtung 16 erleichtert. Durch das Blockierelement 10 wird ein vollständiges Herausziehen des Blasenkatheters 7 aus der Verpackung 2 verhindert und der Katheteraufnahmeraum 3 kann beim Katheterisierungsvorgang nach außen abgedichtet werden. Im Reservoiraufnahmeraum 4 ist ein sachetartiges Reservoir 17 vorgesehen. In dem Reservoir 17 befindet sich ein Aktivierungsmittel zur Aktivierung einer hydrophilen Oberflächenbeschichtung des Blasenkatheters 7, insbesondere des Katheterschafts 9 und der Katheterspitze 8 oder ein Gel als ein Gleitmittel. Das Gleitmittel oder das Aktivierungsmittel kann sich in anderen Ausführungsformen der Erfindung auch direkt (ohne Sachet) im Reservoiraufnahmeraum befinden. Ebenfalls ist eine direkte Befüllung des Reservoiraufnahmeraums mit einem Gleit- oder Aktivierungsmittel denkbar.

In der Verpackung ist eine Aktivierungs- und/oder Gleitmittelführung 20 ausgebildet, die den seitlich neben dem Katheteraufnahmeraum 3 im Bereich des Urinaufnahmeraums 5 angeordneten Reservoiraufnahmeraum 4 mit dem Katheteraufnahmeraum 3 verbindet. Die Aktivierungs- und/oder Gleitmittelführung 20 unterstützt eine in Richtung der Katheterspitze 8 gerichtete Benetzung der Katheteroberfläche mit Aktivierungsmittel und/oder Gleitmittel nach einem Öffnen des Reservoirs 17. Die Aktivierungs- und/oder Gleitmittelführung 20 ist dazu als eine schräg zum Katheteraufnahmeraum 3 in diese Richtung verlaufende Schweißnaht zwischen den die Verpackung 2 ausbildenden Kunststofffolien ausgeführt. Die Aktivierungs- und/oder Gleitmittelführung könnte z.B. auch durch einen entsprechend ausgerichteten, von zwei im Wesentlichen zueinander parallel verlaufenden Schweißnähten gebildeten Kanal als Flüssigkeitsführung ausgebildet sein. Der Winkel zwischen der Aktivierungs- und/oder Gleitmittelführung 20 und der Längsrichtung des Katheteraufnahmeraums 3 ist ein spitzer Winkel. Er beträgt bevorzugt ca. 30 bis 60 Grad.

In einem an die Austrittsstelle 11 angrenzenden Bereich des Katheteraufnahmeraums 3 ist eine Katheteraufnahmeraumverbreiterung 22 vorgesehen. Wenn anstelle des dargestellten Blasenkatheters 7 im Katheteraufnahmeraum 3 ein Tiemannkatheter, also ein Katheter mit gebogener Katheterspitze, vorgehalten werden soll, kann die gebogene Katheterspitze in der Katheteraufnahmeraumverbreiterung 22 ohne Verformung gelagert werden.

Die Handhabung des Katheter-Sets 1 ist folgendermaßen vorgesehen: Das Katheter-Set 1 wird mit geschlossenem oder offenem Verschlusselement 13 in einer sterilen Sekundärpackung geliefert. Das Reservoir 17 mit dem Aktivierungsmittel, z.B. einer sterilen Kochsalzlösung, wird durch Druck geöffnet, so dass das Aktivierungsmittel von dem Reservoiraufnahmeraum 4 in den Katheteraufnahmeraum 3 gelangen und den Blasenkatheter 7 benetzen kann. Durch das Aktivierungsmittel wird eine hydrophile Oberflächenbeschichtung des Blasenkatheters 7 aktiviert und es ergeben sich verbesserte Gleiteigenschaften. Nach einer Wartezeit von ca. 30 Sekunden wird das Verschlusselement 13 geöffnet und der Blasenkatheter 7 kann durch die Führungshülse 12 aus der Verpackung 2 geschoben und über die Harnröhre in die Blase eines Benutzers eingeführt werden. Im Falle eines Gels als Gleitmittel im Reservoir 17 ist das Verschlusselement 13 geöffnet und das Gel wird aus dem Reservoir 17 in Richtung Katheterspitze 8 gedrückt und der Blasenkatheter 7 kann ohne Wartezeit sofort appliziert werden.
Der Urin aus der Blase läuft während des Katheterisierungsvorgangs direkt in die Verpackung 2, bzw. wird durch den Katheterschaft 9 und den Katheteraufnahmeraum 3 in den Urinaufnahmeraum 5 geleitet. Nach Abschluss der Katheterisierung wird der Blasenkatheter 7 aus der Blase und der Harnröhre wieder herausgezogen und zurück in den Katheteraufnahmeraum 3 geschoben und die Verpackung 2 wird mit Hilfe des Verschlusselements 13 wieder dicht verschlossen.

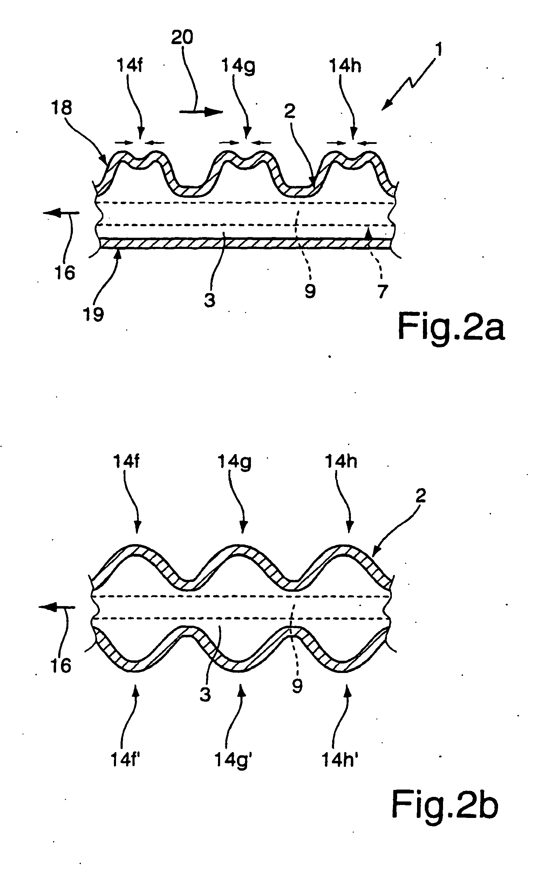

In Figur 2a ist ein Längsschnitt durch das in Figur 1 gezeigte Katheter-Set 1 im Ausschnitt II dargestellt. Der Längsschnitt verläuft entlang der axialen Erstreckung des Katheteraufnahmeraums 3 und des in ihn eingelegten Blasenkatheters 7. Die den Blasenkatheter 7 umhüllende bzw. umgebende Verpackung 2 weist an einer Oberseite 18 Führungselemente 14f, 14g und 14h auf. Durch die Aneinanderreihung der Führungselemente 14f, 14g und 14h erhält die Oberseite 18 eine wellige Kontur, wohingegen eine gegenüberliegende Unterseite 19 der Verpackung 2 glatt ausgeführt ist. Um den Blasenkatheter 7 in Ausführrichtung 16 aus dem Katheteraufnahmeraum 3 zu schieben, wird das flexible Material der Verpackung 2 entlang des Katheterschafts 9 in mehrfacher Wiederholung in einem Abschnitt zwischen beiden an der Verpackung 2 und dem Katheterschaft 9 angreifenden Händen gerafft bzw. verdichtet (mit Pfeilen angedeutet) und anschließend in einer der Ausführrichtung 16 entgegengesetzten Rückschieberichtung 20 gelängt bzw. zurückgeschoben. Das Verschieben der Verpackung 2 findet jeweils gegenüber einem ortsfesten Punkt statt, der durch eine der beiden Hände definiert wird. Die Konturen der Oberseite 18 und der Unterseite 19 der Verpackung 2 werden dabei verändert, wobei die Faltengebung an der Oberseite 18 durch die Führungselemente 14f, 14g und 14h vergleichbar einem Raffband vorgegeben ist. Dadurch werden allzu große Materialverdichtungen, welche sich nur schwer entlang des Katheterschafts 9 verschieben lassen, weitestgehend vermieden.

Der in Figur 2b gezeigte Längsschnitt unterscheidet sich von dem in Figur 2a gezeigten Schnitt darin, dass sowohl an der Oberseite 18 als auch an der Unterseite 19 der Verpackung 2 Führungselemente 14f, 14f', 14g, 14g',... vorgesehen sind. Die Führungselemente 14f, 14f, 14g, 14g',... bilden auf der Ober- und der Unterseite 18, 19 jeweils eine unebene, wellenartige Kontur aus. Beim Bewegen des Blasenkatheters 7 in Ausführrichtung 16 kann beidseits des Katheteraufnahmeraums 3, d.h. sowohl an der Oberseite 18 als auch an der Unterseite 19, der Katheterschaft 9 von einem Benutzer in abrutschsicherer Weise ergriffen und das Material der Verpackung 2 in kontrollierter Weise verschoben werden.

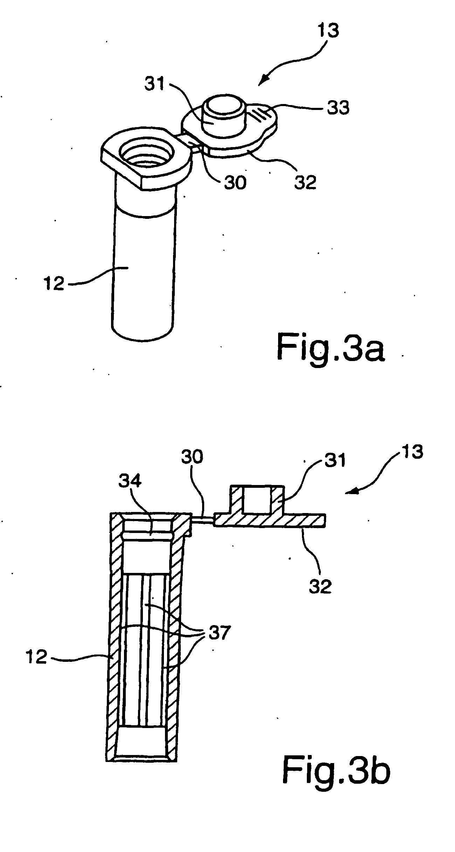

In den Figuren 3a und 3b ist das als Verschlussklappe ausgebildete und an der Führungshülse 12 angeformte Verschlusselement 13 des erfindungsgemäßen Katheter-Sets aus Figur 1 dargestellt. In Figur 3a ist eine dreidimensionale Darstellung gezeigt und in Figur 3b ist ein Längsschnitt gezeigt. Die Verschlussklappe und die Führungshülse 12 sind einstückig aus Kunststoff hergestellt. Um ein Auf- und Zuklappen der Verschlussklappe zu ermöglichen, ist diese über einen flexiblen Kunststoffstreifen 30 mit der Führungshülse 12 verbunden. Die Verschlussklappe wird von einem Kunststoffring 31 und einem über die radiale Außenabmessung der Führungshülse 12 hinausstehenden Deckelteil 32 ausgebildet. Dadurch, dass das Deckelteil 32 über die radiale Außenabmessung der Führungshülse 12 hinaussteht, lässt sich die Verschlussklappe leicht öffnen. Der Deckel 32 weist auf seiner im verschlossenen Zustand der Führungshülse 12 zugewandten Unterseite eine Riffelung 33 zur Verbesserung seiner Griffigkeit auf. Der Kunststoffring 31 bildet einen Pfropfen zum Verschließen der Austrittsstelle 11 aus. Da der Kunststoffring 31 beim Verschließen der Führungshülse 12 radial leicht zusammengepresst wird, ergibt sich eine sehr gute Dichtigkeit gegen Ausfließen von Harn. Eine umlaufende Dichtlippe 34 ist an der Innenoberfläche der Führungshülse 12 angeordnet. Da diese Dichtlippe 34 den Innendurchmesser der Führungshülse 12 im Bereich, in dem der Kunststoffring zum Verschließen in die Führungshülse 12 eingesteckt wird, verengt, erleichtert die Dichtlippe 34 das radiale Zusammenpressen des Kunststoffrings 31 beim Verschließen der Führungshülse 12. Eine entsprechende Dichtlippe kann auch radial umlaufend am Pfropfen vorgesehen sein.
In Figur 3b sind an der Innenoberfläche der Führungshülse 12 in Katheteraustrittsrichtung verlaufende Führungsrippen 37 zur zentrierten Führung des Blasenkatheters bei dessen Applikation zu erkennen.

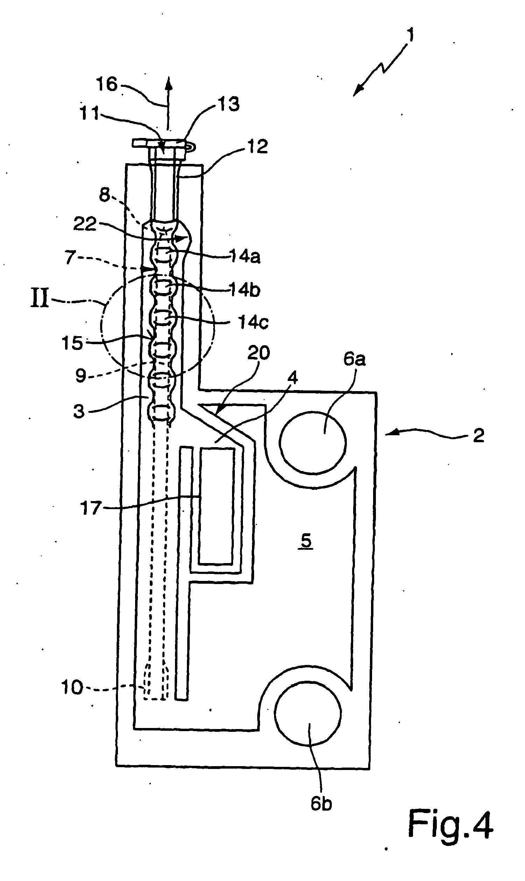

Figur 4 zeigt das erfindungsgemäße Katheter-Set mit einer veränderten äußeren Verpackungsform. Der Urinaufnahmeraum 5 erstreckt sich nicht über die gesamte Länge des Katheteraufnahmeraums und bietet somit gegenüber der Ausführungsform aus Fig. 1 veränderte Applikationsvorteile.

Vorgeschlagen wird ein Katheter-Set 1 umfassend eine aus einem folienartigen Material hergestellte Verpackung 2, in der ein Reservoir 17 mit einem Aktivierungsmittel und/oder einem Gleitmittel vorgesehen ist, und einen Blasenkatheter 7 mit einem flexiblen Katheterschaft 9 und einer Katheterspitze 8, wobei die Verpackung 2 einen im Wesentlichen röhrenförmigen Katheteraufnahmeraum 3 aufweist, in den der Blasenkatheter 7 eingelegt ist, wobei an dem der Katheterspitze 8 zugeordneten Ende des Katheteraufnahmeraums 3 eine Austrittsstelle 11 für den Blasenkatheter 7 vorgesehen ist und wobei das Reservoir 17 an einer Längsseite des Katheteraufnahmeraums 3 am Katheteraufnahmeraum 3 angeordnet ist.
In der Verpackung 2 ist eine in Richtung der Katheterspitze 8 gerichtete Aktivierungs- und/oder Gleitmittelführung 20, zur Unterstützung einer in Katheterspitzenrichtung gerichteten Benetzung der Oberfläche des Blasenkatheters 7 mit dem Aktivierungsmittel und/oder Gleitmittel aus dem Reservoir 17, ausgebildet, die einen in der Verpackung 2 ausgebildeten Reservoiraufnahmeraum 4, in dem das Reservoir 17 angeordnet ist, mit dem Katheteraufnahmeraum 3 verbindet.

Die Erfindung beschränkt sich nicht auf die vorstehend angegebenen Ausführungsbeispiele. Vielmehr ist eine Anzahl von Varianten denkbar, welche auch bei grundsätzlich anders gearteter Ausführung von den Merkmalen der Erfindung Gebrauch machen.

## Patentansprüche

1. Katheter-Set (1) umfassend
eine aus einem folienartigen Material hergestellte Verpackung (2), in der ein Reservoir (17) mit einem Aktivierungsmittel und/oder einem Gleitmittel vorgesehen ist, und einen Blasenkatheter (7) mit einem flexiblen Katheterschaft (9) und einer Katheterspitze (8),
wobei die Verpackung (2) einen im Wesentlichen röhrenförmigen Katheteraufnahmeraum (3) aufweist, in den der Blasenkatheter (7) eingelegt ist,
wobei an dem der Katheterspitze (8) zugeordneten Ende des Katheteraufnahmeraums (3) eine Austrittsstelle (11) für den Blasenkatheter (7) vorgesehen ist,
**dadurch gekennzeichnet, dass**
das Reservoir (17) an einer Längsseite des Katheteraufnahmeraums (3) am Katheteraufnahmeraum (3) angeordnet ist
in der Verpackung (2) eine in Richtung der Katheterspitze (8) gerichtete Aktivierungs- und/oder Gleitmittelführung (20), zur Unterstützung einer in Katheterspitzenrichtung gerichteten Benetzung der Oberfläche des Blasenkatheters (7) mit dem Aktivierungsmittel und/oder Gleitmittel aus dem Reservoir (17), ausgebildet ist,
die einen in der Verpackung (2) ausgebildeten Reservoiraufnahmeraum (4), in dem das Reservoir (17) oder das Gleitmittel-/Aktivierungsmittel direkt angeordnet ist, mit dem Katheteraufnahmeraum (3) verbindet, und die Aktivierungs- und/oder Gleitmittelführung (20) als eine schräg zum Katheteraufnahmeraum (3) in Richtung der Katheterspitze (8) geneigte, bevorzugt von einer Foliennaht ausgebildete, Flüssigkeitsführung ausgeführt ist.

2. Katheter-Set nach Anspruch 1, **dadurch gekennzeichnet, dass** das Reservoir (17) in einem Bereich in der Mitte der Längsausdehnung des Katheteraufnahmeraums (3) in der Verpackung (2) angeordnet ist, bevorzugt wobei der Katheteraufnahmeraum (3) komplett oder teilweise in Längsrichtung der Verpackung (2) verläuft und die Verpackung (2), wie der Urinaufnahmeraum (5), im Wesentlichen rechteckförmig ausgebildet ist.

3. Katheter-Set nach einem der vorhergehenden Ansprüche, **dadurch gekennzeichnet, dass** in zumindest einem Längenabschnitt des Katheteraufnahmeraums (3) Führungselemente (14a, 14b, ...) an der Außenoberfläche (15) der Verpackung (2) vorgesehen sind und
dass die Führungselemente (14a, 14b, ...) in Längsrichtung des Katheteraufnahmeraums (3) der Verpackung (2) dem Verlauf des Katheteraufnahmeraums (3) zumindest teilweise folgen.

4. Katheter-Set nach Anspruch 3, **dadurch gekennzeichnet, dass** die Führungselemente (14a, 14b, ...) im Bereich der Austrittsstelle (11) vorgesehen sind.

5. Katheter-Set nach Anspruch 3, **dadurch gekennzeichnet, dass** sich die Führungselemente (14a, 14b, ...) über die gesamte Länge des Katheteraufnahmeraums (3) erstrecken.

6. Katheter-Set nach einem der Ansprüche 3 bis 5, **dadurch gekennzeichnet, dass** die Führungselemente (14a, 14b, ...) äquidistant, bevorzugt mit fingerbreitem Abstand, angeordnet sind.

7. Katheter-Set nach einem der Ansprüche 3 bis 6, **dadurch gekennzeichnet, dass** die Führungselemente (14a, 14b, ...) an der Oberseite (18) und der Unterseite (19) der Verpackung (2) vorgesehen sind.

8. Katheter-Set nach einem der Ansprüche 3 bis 7, **dadurch gekennzeichnet, dass** die Führungselemente (14a, 14b, ...) als Raffungen, Riffelungen, Noppen und/oder Antirutschelemente ausgebildet sind.

9. Katheter-Set nach einem der vorhergehenden Ansprüche, **dadurch gekennzeichnet, dass** der Katheteraufnahmeraum (3) als Teil eines Urinaufnahmeraums (5) ausgeführt ist und an der Austrittsstelle (11) ein wiederverschließbares Verschlusselement (13) vorgesehen ist.

10. Katheter-Set nach Anspruch 9, **dadurch gekennzeichnet, dass** der Katheteraufnahmeraum (3) eine an die Austrittsstelle (11) angrenzende Führungshülse (12) aufweist, wobei das Verschlusselement (13) als Verschlussklappe an der Führungshülse (12) ausgebildet ist.

11. Katheter-Set nach Anspruch 10, **dadurch gekennzeichnet, dass** die Verschlussklappe und die Führungshülse (12) einstückig aus Kunststoff hergestellt sind, wobei die Verschlussklappe und die Führungshülse (12) über mindestens einen flexiblen Kunststoffstreifen (30) miteinander verbunden sind.

12. Katheter-Set nach einem der Ansprüche 10 oder 11, **dadurch gekennzeichnet, dass** die Führungshülse (12) an ihrer Innenoberfläche in Katheterausführrichtung (16) verlaufende Führungsrippen (37) aufweist.

13. Katheter-Set nach einem der Ansprüche 10 bis 12, **dadurch gekennzeichnet, dass** die Führungshülse (12) und/oder das Verschlusselement (13) eine umlaufende Dichtlippe (34) aufweisen.

14. Katheter-Set nach einem der vorhergehenden Ansprüche, **dadurch gekennzeichnet, dass** in der Verpackung (2) eine oder mehrere Grifföffnungen (6a, 6b) ausgebildet sind.

15. Katheter-Set nach einem der vorhergehenden Ansprüche, **dadurch gekennzeichnet, dass** in einem an die Austrittsstelle (11) angrenzenden Bereich des Katheteraufnahmeraums (3) eine Katheteraufnahmeraumverbreiterung (22) vorgesehen ist.

## Claims

1. A catheter set (1) comprising
a package (2) produced from a foil like material and having provided therein a reservoir (17) containing an activating agent and/or a lubricant, and a urinal catheter (7) having a flexible catheter shaft (9) and a catheter tip (8),
wherein the package (2) comprises a substantially tubular catheter storage space (3) having the urinal catheter (7) inserted therein,
wherein an exit location (11) for the urinal catheter (7) is provided at the end of the catheter storage space (3) associated with the catheter tip (8),
**characterized in that**
the reservoir (17) is arranged on the catheter storage space (3) at a longitudinal side of said catheter storage space (3), the package (2) has formed therein an activating agent and/or lubricant guide (20) directed towards the catheter tip (8) and used for supporting a catheter wetting process in which the surface of the urinal catheter (7) is wetted in the direction of the catheter tip with the activating agent and/or lubricant from the reservoir (17), said activating agent and/or lubricant guide (20) interconnecting a reservoir storage space (4), which is formed in the package (2) and in which the reservoir (17) or the lubricant/activating agent is directly arranged, and the catheter storage space (3), and the activating agent and/or lubricant guide (20) is configured as a fluid guide which is inclined at an oblique angle relative to the catheter storage space (3) in the direction of the catheter tip (8) and which is preferably defined by a foil seam.

2. A catheter set according to claim 1, **characterized in that** the reservoir (17) is arranged in the package (2) in an area in the middle of the length of the catheter storage space (3), the catheter storage space (3) extending preferably fully or in part in the longitudinal direction of the package (2), and the package (2) being, like the urine storage space (5), substantially rectangular.

3. A catheter set according to one of the preceding claims, **characterized in that** guide elements (14a, 14b, ...) are provided on the outer surface (15) of the package (2) in at least one longitudinal section of the catheter storage space (3), and that the guide elements (14a, 14b, ...) follow the profile of the catheter storage space (3) at least partially in the longitudinal direction of the catheter storage space (3) of the package (2).

4. A catheter set according to claim 3, **characterized in that** the guide elements (14a, 14b, ...) are provided in the area of the exit location (11).

5. A catheter set according to claim 3, **characterized in that** the guide elements (14a, 14b, ...) extend over the whole length of the catheter storage space (3).

6. A catheter set according to one of the claims 3 to 5, **characterized in that** the guide elements (14a, 14b, ...) are arranged in an equidistant fashion, preferably at spacings corresponding to the width of a finger.

7. A catheter set according to one of the claims 3 to 6, **characterized in that** the guide elements (14a, 14b, ...) are provided on the upper side (18) and on the lower side (19) of the package (2).

8. A catheter set according to one of the claims 3 to 7, **characterized in that** the guide elements (14a, 14b, ...) are implemented as gathers, corrugations, knobs and/or anti-skid elements.

9. A catheter set according to one of the preceding claims, **characterized in that** the catheter storage space (3) is configured as part of a urine storage space (5) and that a reclosable closure element (13) is provided on the exit location (11).

10. A catheter set according to claim 9, **characterized in that** the catheter storage space (3) is provided with a guide sleeve (12) adjoining the exit location (11), the closure element (13) being implemented as a closure flap on said guide sleeve (12).

11. A catheter set according to claim 10, **characterized in that** the closure flap and the guide sleeve (12) are produced from plastic material in one piece, said closure flap and said guide sleeve (12) being connected to one another by at least one strip of flexible plastic material (30).

12. A catheter set according to one of the claims 10 or 11, **characterized in that** the inner surface of the guide sleeve (12) is provided with guide ribs (37) extending in the catheter removal direction (16).

13. A catheter set according to one of the claims 10 to 12, **characterized in that** the guide sleeve (12) and/or the closure element (13) are provided with a circumferentially extending sealing lip (34).

14. A catheter set according to one of the preceding claims, **characterized in that** the package (2) is provided with one or more handle openings (6a, 6b).

15. A catheter set according to one of the preceding claims, **characterized in that** a catheter storage space enlargement (22) is provided in an area of the catheter storage space (3) adjoining the exit location (11).

## Revendications

1. Ensemble de cathéter (1) comprenant
un emballage (2) qui est fabriqué à partir d'un matériau en forme de film et dans lequel est prévu un réservoir (17) avec un agent d'activation et/ou un lubrifiant, et une sonde vésicale (7) avec une tige de cathéter flexible (9) et une pointe de cathéter (8),
l'emballage (2) comportant un logement de cathéter (3) globalement tubulaire dans lequel est placée la sonde vésicale (7), et
une sortie (11) pour la sonde vésicale (7) étant prévue à l'extrémité du logement de cathéter (3) associée à la pointe de cathéter (8),
**caractérisé en ce que** le réservoir (17) est disposé sur le logement de cathéter (3) sur un côté longitudinal dudit logement de cathéter (3),
il est prévu dans l'emballage (2) un conduit d'agent d'activation et/ou de lubrifiant (20) qui est dirigé vers la pointe de cathéter (8), qui est destiné à renforcer un mouillage, dans le sens de ladite pointe, de la surface de la sonde vésicale (7) avec l'agent d'activation et/ou le lubrifiant à partir du réservoir (17),
et qui relie au logement de cathéter (3) un logement de réservoir (4) qui est formé dans l'emballage (2) et dans lequel le réservoir (17) ou le lubrifiant/l'agent d'activation est disposé directement, et
le conduit d'agent d'activation et/ou de lubrifiant (20) est conçu comme un conduit de liquide qui est incliné par rapport au logement de cathéter (3) en direction de la pointe de cathéter (8) et qui est de préférence formé par une soudure de film.

2. Ensemble de cathéter selon la revendication 1, **caractérisé en ce que** le réservoir (17) est disposé dans l'emballage (2) dans une zone située au milieu de l'extension longitudinale du logement de cathéter (3), le logement de cathéter (3) s'étendant de préférence complètement ou partiellement dans le sens longitudinal de l'emballage (2), et l'emballage (2) ayant comme le collecteur d'urine (5) une forme globalement rectangulaire.

3. Ensemble de cathéter selon l'une des revendications précédentes, **caractérisé en ce qu'**il est prévu dans au moins un tronçon du logement de cathéter (3) des éléments de guidage (14a, 14b, ...) sur la surface extérieure (15) de l'emballage (2) et
**en ce que** les éléments de guidage (14a, 14b,...), dans le sens longitudinal du logement de cathéter (3) de l'emballage (2), suivent au moins en partie la forme dudit logement de cathéter (3).

4. Ensemble de cathéter selon la revendication 3, **caractérisé en ce que** les éléments de guidage (14a, 14b, ...) sont prévus dans la zone de la sortie (11).

5. Ensemble de cathéter selon la revendication 3, **caractérisé en ce que** les éléments de guidage (14a, 14b, ...) s'étendent sur toute la longueur du logement de cathéter (3).

6. Ensemble de cathéter selon l'une des revendications 3 à 5, **caractérisé en ce que** les éléments de guidage (14a, 14b,...) sont équidistants, et présentent de préférence un écartement de la largeur d'un doigt.

7. Ensemble de cathéter selon l'une des revendications 3 à 6, **caractérisé en ce que** les éléments de guidage (14a, 14b,...) sont prévus sur le côté supérieur (18) et sur le côté inférieur (19) de l'emballage (2).

8. Ensemble de cathéter selon l'une des revendications 3 à 7, **caractérisé en ce que** les éléments de guidage (14a, 14b,...) sont conçus comme un striage, un cannelage, des bosses et/ou des éléments antidérapants.

9. Ensemble de cathéter selon l'une des revendications précédentes, **caractérisé en ce que** le logement de cathéter (3) est conçu comme une partie d'un collecteur d'urine (5), et il est prévu au niveau de la sortie (11) un élément de fermeture (13) apte à être refermé.

10. Ensemble de cathéter selon la revendication 9, **caractérisé en ce que** le logement de cathéter (3) présente un manchon de guidage (12) qui est voisin de la sortie (11), l'élément de fermeture (13) étant conçu comme un couvercle à charnière sur le manchon de guidage (12).

11. Ensemble de cathéter selon la revendication 10, **caractérisé en ce que** le couvercle à charnière et le manchon de guidage (12) sont fabriqués d'une seule pièce en matière plastique, le couvercle et le manchon (12) étant reliés par au moins une bande de matière plastique flexible (30).

12. Ensemble de cathéter selon l'une des revendications 10 ou 11, **caractérisé en ce que** le manchon de guidage (12) présente sur sa surface intérieure des nervures de guidage (37) qui s'étendent dans le sens d'extraction (16) du cathéter.

13. Ensemble de cathéter selon l'une des revendications 10 à 12, **caractérisé en ce que** le manchon de guidage (12) et/ou l'élément de fermeture (13) présentent une lèvre d'étanchéité circulaire (34).

14. Ensemble de cathéter selon l'une des revendications précédentes, **caractérisé en ce qu'**une ou plusieurs ouvertures de préhension (6a, 6b) sont formées dans l'emballage (2).

15. Ensemble de cathéter selon l'une des revendications précédentes, **caractérisé en ce qu'**un élargissement de logement de cathéter (22) est prévu dans une zone du logement de cathéter (3) qui est voisine de la sortie (11).
